# EUROPEAN PATENT SPECIFICATION

(11) **EP 0 930 888 B1**
(45) Date of publication and mention of the grant of the patent: **05.06.2002**
(21) Application number: 97911191.1
(22) Date of filing: 07.10.1997
(51) Int. Cl.: A61K 35/78, A61K 7/48

(54) **PHARMACEUTICAL AND COSMETIC ANTIACNE FORMULATIONS CONTAINING PLANT EXTRACTS (KRAMERIA TRIANDRA OR MESUA FERREA)**
PHARMAZEUTISCHE UND KOSMETISCHE ANTIAKNEZUSAMMENSETZUNGEN, DIE PFLANZENEXTRAKTE (KRAMERIA TRIANDRA ODER MESUA FERREA) ENTHALTEN
FORMULATIONS PHARMACEUTIQUES ET COSMETIQUES CONTRE L'ACNE CONTENANT DES EXTRAITS DE PLANTES (KRAMERIA TRIANDRA OU MESUA FERREA)

(30) Priority: 17.10.1996 IT MI962149
(43) Date of publication of application: 28.07.1999
(73) Proprietor: INDENA S.p.A., 20139 Milano (IT)
(72) Inventor: BOMBARDELLI, Ezio, I-20141 Milano (IT); CRISTONI, Aldo, I-20139 Milano (IT); MORAZZONI, Paolo, I-20139 Milano (IT); SEGHIZZI, Roberto, I-20139 Milano (IT)
(74) Representative: Minoja, Fabrizio, Dr.
(86) International application number: EP9705510
(87) International publication number: WO9817293

(56) References cited:
- EP-A- 0 283 713
- EP-A- 0 304 603
- EP-A- 0 464 297
- GB-A- 2 274 058
- US-A- 4 886 667
- PATENT ABSTRACTS OF JAPAN vol. 096, no. 005, 31 May 1996 & JP 08 012565 A (SHISEIDO CO LTD), 16 January 1996,

## Description

The present invention relates to pharmaceutical and cosmetic formulations with antiacne activity containing:
a) an antimicrobial hydrophilic extract of Krameria sp., or a pure compound from said extract and/or an antimicrobial lipophilic extract of Mesua ferrea;
b) ximeninic acid and/or lauric acid;
c) an antiinflammatory saponin extracted from Olax dissitiflora, Aesculum hippocastanum, Centella asiatica, Terminalia sericea, Glycyrrhiza glabra.

### PRIOR ART

It is known from literature (Martindale, "The extrapharmacopeia", 28th Ed. (1982); Cannizaro, Boll. Soc. Ital. Biol. Sperim. 1,22, 1964; V. Hoppe, Drogenkunde Bdl Walter De Gruyter Ed., 1975; and British patent 2.184.353 A) that the extracts obtained by extraction with chlorinated solvents, aliphatic ethers and ketones as well as aliphatic and aromatic esters, from the roots, the bark of the trunk and the leaves of different species of Krameria, preferably Krameria triandra, enriched in neolignanes (particularly Eupomatenoid 6 and 2-(2,4-dihydroxyphenyl)-5-propenylbenzofuran) have antimicrobial activity against gram +, gram - bacteria, fungi and anaerobic strains.

These extracts, although obtainable by extraction even with protic solvents, are naturally hydrophilic due to the phenolic character of their active components.

The pure components Eupomatenoid 6 and 2-(2,4-dihydroxyphenyl)-5-propenylbenzofuran can be recovered from these extracts using chromatographic techniques on silica gel, as reported in EP 0 464 297 B1.

From the flowers, particularly from the buds, of Mesua ferrea, extraction with aprotic solvents such as hexane,. methylene chloride or better with carbon dioxide in hyper-critic conditions, for example extracting the vegetable material under pressures ranging from 110 to 260 bar, mainly at 200 bar and at temperatures ranging from 35 to 65°C, preferably at 45°C, yields a lipophilic extract containing substituted coumarins and xanthones, which results particularly active on gram +, gram - and anaerobic bacterial strains, with an activity comparable to that of the extract prepared from Krameria triandra.

### DISCLOSURE OF THE INVENTION

It has surprisingly been found that the combination of extracts of some plants naturally having an antimicrobial activity, specifically extracts or pure compounds from different species of Krameria, preferably Krameria triandra, and from Mesua ferrea, with substances such as ximeninic acid and with saponins such as those obtainable from Olax dissitiflora, Aesculus hippocastanum, Centella asiatica, Terminalia sericea, Glycyrrhiza glabra, with or without the addition of low molecular fatty acids such as lauric acid, myristic, isomyristic etc., is highly active in the treatment of acne.

The combination of this mixture of antimicrobial agents with ximeninic acid and with natural compounds having antiinflammatory activity, allows to obtain a nearly complete disappearance of the acne manifestations and an effective prevention of the acne development. Particularly effective proved a combination of the antimicrobial agents with ximeninic acid, lauric acid (one of the main constituents of the lipophilic extract of Serenoa repens) and a saponin selected from those extractable from Olax dissitiflora, Aesculus hippocastanum, Centella asiatica, Terminalia sericea, Glycyrrhiza glabra, preferably those extracted from Aesculus hippocastanum and Centella asiatica.

### PHARMACOLOGICAL STUDIES

Among the various combinations tested, those which showed the best clinical results contain 0.1 to 0.5% of extract of Krameria triandra, preferably 0,2%, 0.1 to 0.5% of extract of Mesua ferrea, preferably 0.25%, 0.2 to 1% of ximeninic acid, preferably 0.6%, 0.1 to 0.4% of lauric acid, preferably 0.2%, and 0.35 to 1% of escin, preferably 0.5%.

The antiacne activity has been tested on 30 patients of both sexes, of the age of 18 to 35 years. Patients have been treated with 0.5 cm of the formulation twice a day for 45 consecutive days. The untreated part of the face has been used as the control, At the end of the treatment the number of papules, pustoles and comedos (A), the sebum concentration (B) and the presence of *Propionibacterium acnes* in the exudate from the pustoles and comedos (C) have been evaluated.

The results obtained are reported in the following Table.

**Table**

| **TREATMENT** | **% REDUCTION** | | |
|---|---|---|---|
| | **A** | **B** | **C** |
| **None** | 5 | 2.3 | 0 |
| Formulation 1 (Ex. IV) | 70 | 13.1 | 80 |
| Formulation 2 (Ex. V) | 50 | 12.4 | 70 |
| Formulation 3 * | 20 | 4 | 20 |

| | | | |
|---|---|---|---|
| * formulation containing ximeninic acid and escin β-sitosterol (EP 283713). | | | |

The present invention, therefore, relates to pharmaceutical and cosmetic formulations with antiacne activity, which can be administered topically (gels, creams, lotions, milks and solid preparations) containing the combinations described above. Said formulations will be prepared according to conventional methods well known in pharmaceutical technique, as those described in "Remington's Pharmaceutical Handbook", Mack Publishing Co., N.Y., USA, together with suitable excipients, in particular antioxidants.

The following examples illustrate the invention in further detail.

### Example I - Preparation of the lipophilic extract of Mesua ferrea

1 kg of finely ground buds of Mesua ferrea is extracted in a 5 1 extractor with CO₂ in hyper-critic conditions; a first extraction is carried out at 34°C and 90 bars of pressure, using about 25 1 of carbon dioxide; the resulting extract is discarded and the vegetable material is subjected to a second extraction, increasing temperature to 45°C and pressure to 220 bar. 15.6 g of a very thick oil of orange colour are obtained. This oil can be used directly as such or subjected to further fractionations by means of conventional chemical separations. The extract is preferably used as such.

### Example II - Preparation of the extract of Krameria triandra

2 kg of bark of the root of Krameria triandra, after grinding, are extracted three times with 5 1 each of acetone; the combined extracts are concentrated to small volume and the concentrate is taken up in 0.8 1 of acetone:water 1:1 (v/v). The resulting suspension is extracted twice with 0.5 l of methylene chloride, the chloromethylene phase is dried over anhydrous sodium sulfate, then concentrated to dryness. 85 g of a reddish solid are obtained, containing about 26% of Eupomatenoid 6 and 14% of 2-(2,4-dihydroxyphenyl)-5-propenylbenzofuran.

### Example III - Preparation of the extract of Mesua ferrea with hexane

1 kg of finely ground buds of Mesua ferrea is extracted 4 times with 3 1 of hexane under reflux; the combined hexane extracts are decolourized with active charcoal (5 g) and concentrated under vacuum to an oil. 14.5 g of extract are obtained, which can be used without further fractionation.

### Example IV - Preparation of a Water/Silicon emulsion

| | |
|---|---|
| Mesua Ferrea | 0.25 g |
| Krameria triandra | 0.2 g |
| Ximeninic acid | 0.60 g |
| Lauric acid | 0.20 g |
| 18-β Glycyrrhetic acid | 0.50 g |
| Propylene glycol | 5.0 g |
| Sodium Coccoylglutamate 25% sol. | 2.0 g |
| Mineral oil and lanolin alcohols | 2.5 g |
| Octyl Octanoate | 5.0 g |
| Octododecyl Myristate | 7.5 g |
| Cetyl Dimethycone Copolyol | 2.5 g |
| Tetramer Cyclomethicone | 5.0 g |
| Sodium Chloride | 2.0 g |
| Glycerin | 2.5 g |
| Imidurea | 0.3 g |
| Methylparaben | 0.1 g |
| Purified water q.s. to | 100.0 g. |

### Example V - Preparation of a Oil/Water emulsion

| | |
|---|---|
| Krameria Triandria | 0.20 g |
| Bscin β-Sitosterol fitosoma^{R} | 0.50 g |
| Ximeninic acid | 0.50 g |
| Lauric acid | 0.20 g |
| Gliceryl Monostearate | 3.00 g |
| Alkyl C₁₂-Benzoate | 7.00 g |
| Silicon oil | 0.50 g |
| Carbomer | 0.50 g |
| Acrylate/C₁₀₋₃₀ Alkylacrylate Crosspolymer | 0.20 g |
| Sodium Lauryl Sulfate | 1.00 g |
| Propylene glycol | 5.00 g |
| Sodium hydroxide 10% sol. | 1.50 g |
| Imidurea | 0.3 g |
| Methylparaben | 0.1 g |
| Purified water q.s. to | 100.00 g. |

### Example VI - Oil/Water emulsion

| | |
|---|---|
| Krameria Triandria | 0.25 g |
| Escin β-Sitosterol fitosoma^{R} | 0.50 g |
| Lauric acid | 0.20 g |
| Polyglyceryl 8-pentastearate and behenyl alcohol and sodium stearoyl lactylate (Nikkhomulese 41 - Nikkho) | 2.50 g |
| Behenyl alcohol | 1.50 g |
| Squalane | 7.00 g |
| Trioctanoine | 6.00 g |
| PPG-12/SMDI | 2.00 g |
| Butenyl glycol | 5.00 g |
| Xanthan gum | 0.30 g |
| Preservatives | q.s. |
| Purified water q.s. to | 100.00 g. |

## Claims

1. Pharmaceutical and cosmetic formulations with antiacne activity containing:
a) an antimicrobial hydrophilic extract of Krameria sp., or a pure compound from said extract selected from the group consisting of Eupomatenoid 6 and 2-(2,4-dihydroxyphenyl)-5-propenylbenzofuran and/or an antimicrobial lipophilic extract of Mesua ferrea and
b) ximeninic acid and/or lauric acid and
c) an antiinflammatory saponin extracted from Olax dissitiflora, Aesculus hippocastanum, Centella asiatica, Terminalia sericea, Glycyrrhiza glabra.

2. Formulations according to claim 1, containing:
- 0.1 to 0.5% of extract of Krameria triandra,
- 0.1 to 0.5% of extract of Mesua ferrea,
- 0.2 to 1% of ximeninic acid,
- 0.1 to 0.4% of lauric acid
- 0.35 to 1% of escin.

3. Formulations according to claim 2, containing:
- 0.2% of extract of Krameria triandra,
- 0.25% of extract of Mesua ferrea,
- 0.6% of ximeninic acid,
- 0.2% of lauric acid,
- 0.5% of escin.

4. The use of:
a) an antimicrobial hydrophilic extract of Krameria sp., or a pure compound from said extract selected from the group consisting of Eupomatenoid 6 and 2-(2,4-dihydroxyphenyl)-5-propenylbenzofuran and/or an antimicrobial lipophilic extract of Mesua ferrea and
b) ximeninic acid and/or lauric acid and
c) an antiinflammatory saponin extracted from Olax dissitiflora, Aesculus hippocastanum, Centella asiatica, Terminalia sericea, Glycyrrhiza glabra,
for the preparation of a medicament with antiacne activity.

## Patentansprüche

1. Pharmazeutische und kosmetische Formulierungen mit Antiaknewirkung, enthaltend:
a) einen antimikrobiellen hydrophilen Extrakt von Krameria sp., oder eine reine Verbindung aus diesem Extrakt, ausgewählt aus der Gruppe, bestehend aus Eupomatenoid 6 und 2-(2,4-Dihydroxyphenyl)-5-propenylbenzofuran und/oder einen antimikrobiellen lipophilen Extrakt von Mesua ferrea und
b) Ximeninsäure und/oder Laurinsäure und
c) ein entzündungshemmendes Saponin, extrahiert aus Olax dissitiflora, Aesculus hippocastanum, Centella asiatica, Terminalia sericea, Glycyrrhiza glabra.

2. Formulierungen nach Anspruch 1, enthaltend:
- 0,1 bis 0,5 % Extrakt von Krameria triandra,
- 0,1 bis 0,5 % Extrakt von Mesua ferrea,
- 0,2 bis 1 % Ximeninsäure,
- 0,1 bis 0,4 % Laurinsäure,
- 0,35 bis 1 % Äscin.

3. Formulierungen nach Anspruch 2, enthaltend:
- 0,2 % Extrakt von Krameria triandra,
- 0,25 % Extrakt von Mesua ferrea,
- 0,6 % Ximeninsäure,
- 0,2 % Laurinsäure,
- 0,5 % Äscin.

4. Verwendung von:
a) einem antimikrobiellen hydrophilen Extrakt von Krameria sp., oder eine reine Verbindung aus diesem Extrakt, ausgewählt aus der Gruppe, bestehend aus Eupomatenoid 6 und 2-(2,4-Dihydroxyphenyl)-5-propenylbenzofuran und/oder einen antimikrobiellen lipophilen Extrakt von Mesua ferrea und
b) Ximeninsäure und/oder Laurinsäure und
c) einem entzündungshemmenden Saponin, extrahiert aus Olax dissitiflora, Aesculus hippocastanum, Centella asiatica, Terminalia sericea, Glycyrrhiza glabra,
für die Herstellung eines Arzneimittels mit Antiaknewirkung.

## Revendications

1. Formulations pharmaceutiques et cosmétiques ayant une activité anti-acnée contenant :
a) un extrait hydrophile antimicrobien de Krameria sp., ou un composé pur dudit extrait choisi dans le groupe constitué par l'Eupomaténoïde 6 et le 2-(2,4-dihydroxyphényl)-5-propénylbenzofuranne et/ou un extrait lipophile antimicrobien de Mesua ferrea et
b) de l'acide ximéninique et/ou de l'acide laurique et
c) une saponine antiinflammatoire extraite à partir de Olax dissitiflora, d'Aesculus hippocastanum, de Centella asiatica, de Terminalia sericea ou de Glycyrrhiza glabra.

2. Formulations selon la revendication 1, contenant :
- 0,1 à 0,5% d'extrait de Krameria triandra,
- 0,1 à 0,5% d'extrait de Mesua ferrea,
- 0,2 à 1% d'acide ximéninique,
- 0,1 à 0,4% d'acide laurique
- 0,35 à 1% d'escine.

3. Formulations selon la revendication 2, contenant :
- 0,2% d'extrait de Krameria triandra,
- 0,25% d'extrait de Mesua ferrea,
- 0,6% d'acide ximéninique,
- 0,2% d'acide laurique
- 0,5% d'escine.

4. Utilisation :
a) d'un extrait hydrophile antimicrobien de Krameria sp., ou d'un composé pur dudit extrait choisi dans le groupe constitué par l'Eupomaténoide 6 et le 2-(2,4-dihydroxyphényl)-5-propénylbenzofuranne et/ou un extrait lipophile antimicrobien de Mesua ferrea et
b) d'acide ximéninique et/ou d'acide laurique et
c) d'une saponine antiinflammatoire extraite à partir d'Olax dissitiflora, d'Aesculus hippocastanum, de Centella asiatica, de Terminalia sericea ou de Glycyrrhiza glabra,
pour la préparation d'un médicament ayant une activité anti-acnée.
